# FASCICULE DE BREVET EUROPEEN

(11) **EP 1 112 057 B1**
(45) Date de publication et mention de la délivrance du brevet: **14.03.2007**
(21) Numéro de dépôt: 99942962.4
(22) Date de dépôt: 13.09.1999
(51) Int. Cl.: A61K 8/04, A61K 8/64, A61Q 19/00, A61Q 19/08

(54) **UTILISATION COSMETIQUE OU DERMAPHARMACEUTIQUE DE PEPTIDES POUR LA CICATRISATION, L'HYDRATATION ET L'AMELIORATION DE L'ASPECT CUTANE LORS DU VIEILLISSEMENT NATUREL OU ACCELERE (HELIODERMIE, POLLUTION)**
KOSMETISCHE ODER DERMOPHARMAZEUTISCHE VERWENDUNG VON PEPTIDEN ZUR WUNDHEILUNG, HYDRATISIERUNG, VERBESSERUNG DES HAUTAUSSEHENS WÄHREND DER NATÜRLICHEN ODER BESCHLEUNIGTEN ALTERUNG (HELIODERMIE, POLLUTION)
COSMETIC OR DERMOPHARMACEUTICAL USE OF PEPTIDES FOR HEALING, HYDRATING AND IMPROVING SKIN APPEARANCE DURING NATURAL OR INDUCED AGEING (HELIODERMIA, POLLUTION)

(30) Priorité: 15.09.1998 FR 9811533
(43) Date de publication de la demande: 04.07.2001
(73) Titulaire: SEDERMA, 78612 Le Perray en Yvelines Cedex (FR)
(72) Inventeur: LINTNER, Karl, F-78120 Rambouillet (FR)
(74) Mandataire: Laget, Jean-Loup
(86) Numéro de dépôt international: PCT/FR1999/002178
(87) Numéro de publication internationale: WO 2000/015188

(56) Documents cités:
- K. KATAYAMA E.A.: "A Pentapeptide from Type I Procollagen Promotes Extracellular Matrix Production" JOURNAL OF BIOLOGICAL CHEMISTRY, vol. 268, no. 14, 1993, pages 9941-9944, XP002106610 cité dans la demande

## Description

Le vieillissement, notamment celui de la peau, entraîne d'importantes perturbations biochimiques tissulaires intimes qui se concrétisent par des modifications macroscopiques, habituellement jugées disgracieuses, et qui n'ont cessé de préoccuper tant les femmes que les hommes.

La quête du bronzage par les UV naturels, solaires, ou par ceux, artificiels, des *salons de beauté,* est responsable d'un processus de vieillissement cutané bien connu des Dermatologues sous le nom *d'héliodermie* (D^{r} C. Musy-Preault, (1994) *Les Maladies de la peau,* Albin Michel ed., Paris).

D'autres composants de notre mode de vie actuel, tels que les agressions physiques et chimiques de la pollution; la consommation d'alcool et de tabac, favorisent et aggravent les processus de vieillissement.

Par ailleurs, au cours de la vie privée ou professionnelle, la peau, premier rempart de l'organisme vis-à-vis du monde extérieur, est menacée dans son intégrité par de nombreuses agressions ponctuelles comme les coupures, brûlures, réactions inflammatoires. Pour en corriger les dégâts, l'organisme a développé une série de réactions, complexes et imbriquées entre elles: la cicatrisation.

L'industrie cosmétique est en permanence à la recherche de nouveaux ingrédients capables de s'opposer aux effets du vieillissement en général et/ou de favoriser la cicatrisation cutanée.

Pour cela, une des approches possibles consiste à favoriser la restructuration tissulaire par la néo-synthèse des différents éléments constitutifs de la peau. Tout comme le ciment qui assure la cohésion des briques dans un mur et qui lui confère sa solidité, les différents types de collagène et autres mucopolysaccharides, sont les éléments constitutifs du tissu cutané.

Favoriser la synthèse et l'incorporation de ces molécules est certes obligatoire mais pas suffisant en soi. Il faut également *préparer le terrain* en lui donnant une bonne assise sur laquelle les mécanismes de la cicatrisation pourront effectuer des réparations durables. Dans les situations décrites ci-dessus, cette assise est la matrice extracellulaire, qui prend le nom de lame basale lorsqu'elle est située à l'interface de l'épithélium et du tissu conjonctif. L'amélioration ou la reconstruction de la matrice extracellulaire est primordiale car l'on sait maintenant que non seulement cette structure joue *"le rôle de charpente stabilisant la structure physique des tissus"* mais qu'également, elle *"joue un rôle .... dans la régulation du comportement des cellules qui sont à son contact - influant sur leur développement, leur migration, leur prolifération, leur forme et leurs fonctions* (Biologie Moléculaire de la Cellule, 3^{ème} éd. Médecine-Sciences, Flammarion, Paris, page 972). Nous nous sommes donc spécialement intéressés à deux des principaux constituants de cette matrice extracellulaire: les collagènes et les glycosaminoglycanes (également connues sous le nom de GAGs).

Dans le cadre de ce brevet, les effets du vieillissement sur les collagènes et les glycosaminoglycanes peuvent se résumer par:
- La *diminution de la synthèse* de ces molécules par les fibroblastes, diminution due à la conjonction de deux causes: d'une part le taux de renouvellement de ces cellules productrices diminue avec l'âge et, d'autre part, la quantité de molécules sécrétées par ces cellules diminue également.
   Lorsque l'on sait que le collagène représente environ 80% des protéines cutanées, il est facile de comprendre que la moindre diminution de sa concentration tissulaire puisse avoir des conséquences importantes sur les propriétés mécaniques et physiologiques de la peau.
   Les glycosaminoglycanes sont capables de fixer de grandes quantité d'eau. La baisse de leur concentration tissulaire entraîne donc une déshydratation cutanée.
- *L'apparition de modifications structurales* des molécules néo-synthétisées qui conduisent à la réticulation des fibres et donc, à leur rigidification.
   Pour le collagène, les variations des chaînes α modifient la répartition de ses différentes formes. Par exemple, la proportion de collagène de type III augmente dans l'épiderme quand le collagène de type IV s'accumule dans la membrane basale. On constate également l'apparition de réactions, enzymatiques ou non (de type réaction de Maillard), qui créent des liaisons, dites croisées, soit entre deux fibres de collagène, soit entre le collagène lui-même et des molécules de glucose, rigidifiant ainsi les réseaux de fibres de collagène.
   Le vieillissement se traduit sur les glycosaminoglycanes par la synthèse imparfaite de leurs chaînes polysaccharides et par une diminution de leur sulfatation. Plus encore que sur le collagène, les formes radicalaires de l'oxygène dégradent les GAGs de manière irréversible.

La peau perd donc de sa *substance* par la diminution de la quantité de ses constituants, se durcit par la perte d'élasticité des fibres de collagène et par sa déshydratation.

Tout ceci contribue à donner à la peau âgée ses aspects caractéristiques: sécheresse, absence de souplesse, finesse, fragilité, rides plus ou moins nombreuses et plus ou moins profondes.

La cicatrisation quant à elle, fait appel, au moins partiellement, à des besoins semblables puisqu'il lui faut reconstruire, et donc fabriquer de la masse tissulaire; ce qui implique localement, la synthèse accrue des différents constituants cutanés.

Ainsi, tout produit capable d'induire un, ou des, processus augmentant localement la synthèse des collagènes et des glycosaminoglycanes, permettra d'obtenir l'effet recherché par toutes les personnes voulant réduire les stigmates cutanés du vieillissement ainsi que par celles voulant améliorer la cicatrisation, aussi bien dans le temps que dans l'esthétisme et la qualité du résultat.

L'invention faisant l'objet de cette demande de brevet réside dans le fait que nous avons développé un produit qui répond aux critères précédents et que nous en avons démontré l'efficacité *in vitro* et *in vivo,* par des tests scientifiques sophistiqués.

Il est connu que la synthèse de collagène peut être stimulée (*in vitro)*, dans des cultures cellulaires, par le fragment C-terminal du collagène I que constitue le peptide Lys-Thr-Thr-Lys-Ser (Katayama K. et al., *Journal of Biological Chemistry* (1993),**259**:9941-9944.

Par ailleurs, il est possible d'augmenter la synthèse des glycosaminoglycanes cutanés par des extraits végétaux (par exemple, chez le rat: Chithra P. et al., *Journal of Ethnopharmacology* (1998),**59**:179-186).

Notre demande de brevet réside dans la découverte, qu'administrés, seuls ou en association entre eux, par voie topique *in vivo,* et donc dans une approche relevant de la cosmétique, les peptides de formule générale R₁-X-Thr-Thr-Lys-(AA)ₙ-Y et leurs sels avec:
- X représentant un acide aminé basique de forme D ou L (lysine, arginine, histidine, omithine, citrulline, sarcosine, statine),
- (AA)ₙ représentant un enchaînement de n acides aminés naturels ou non, avec n variant de 0 à 5,
- R₁ étant H ou une chaîne d'acide gras, de 2 à 22 carbones, hydroxylée ou non, saturée ou non, linéaire ou ramifiée, soufrée ou non, cyclique ou non, ou un groupement biotinyl, ou un groupement protecteur de type uréthane utilisé en synthèse peptidique tel que les groupements benzyloxycarbonyl (Z), terbutyloxycarbonyl (tBoc), fluorenylmethyloxycarbonyl (Fmoc), allyloxycarbonyl (Alloc)
- Y=OR₂ ou NR₂R₃ avec R₂ et/ou R₃ étant un atome d'hydrogène H ou une chaîne aliphatique ou aromatique de 1 à 22 carbones, hydroxylée ou non, saturée ou non, linéaire ou ramifiée, soufrée ou non, cyclique ou non,
à l'exception des peptides avec R₁=H et X=Lys et Y=OH et avec n=0 ou (AA)ₙ=Ser quand n=1,
sont capables d'augmenter, de manière très importante, la synthèse concomitante du collagène et celle des glycosaminoglycanes et que ce fait permet d'obtenir un effet de synergie car alors, le résultat observé est supérieur à ce que l'on pouvait espérer de l'addition de chacun de ces effets.

En effet, les fibres de collagène nouvellement formées s'imbriquent immédiatement dans le treillis des glycosaminoglycanes de la lame basale nouvellement synthétisée; accélérant ainsi le processus de régénération cutané ainsi que le niveau moyen d'hydratation tissulaire.

Les peptides utilisés avantageusement dans cette optique peuvent être caractérisés en ce que n=1, R₁ est une chaîne d'acide gras de 2 à 22 carbones et Y est OH ou NH₂, et plus précisément avec X = lysine, (AA)ₙ = sérine, R₁ = le groupement palmitoyl et Y = OH.

Les peptides, objets de cette demande de brevet, peuvent être obtenus soit par synthèse chimique classique (en phase hétérogène ou en phase homogène), soit par synthèse enzymatique (Kullman et al., J. Biol. Chem. 1980, 255, 8234) à partir des acides aminés constitutifs ou de leurs dérivés.

La petite taille de ces peptides permet d'en faire la synthèse industrielle, à un coût avantageux. Leur grande activité démontrée en autorise l'utilisation commerciale dans un grand nombre de produits cosmétiques ou dermopharmaceutiques financièrement acceptables.

Les peptides peuvent être obtenus également par fermentation d'une souche de bactéries, modifiées ou non par génie génétique, pour produire les séquences recherchées ou leurs différents fragments.

Enfin, les peptides peuvent être obtenus par extraction de protéines d'origine animale ou végétale, préférentiellement végétale, susceptibles de contenir ces séquences au sein de leur structure, suivie d'une hydrolyse contrôlée, enzymatique ou non, qui libère les fragments peptidiques en question (de séquence X-Thr-Thr-Lys-(AA) préférentiellement Lys-Thr-Thr-Lys-Ser), de taille moyenne comprise entre 300 et 2000 daltons, avec la stipulation que les fragments libérés correspondent à la séquence peptidique précédente dans les plantes qui sont susceptibles de contenir ces séquences au sein de leur structure. L'hydrolyse ménagée permet de dégager ces fragments peptidiques.

Pour réaliser l'invention, il est possible, mais non nécessaire, soit d'extraire les protéines concernées d'abord et de les hydrolyser ensuite, soit d'effectuer l'hydrolyse d'abord sur un extrait brut et de purifier les fragments peptidiques ensuite. On peut également utiliser l'hydrolysât sans en extraire les fragments peptidiques en question, en s'assurant toutefois de l'arrêt de la réaction enzymatique d'hydrolyse à temps et de doser la présence des peptides en question par des moyens analytiques appropriés (traçage par radioactivité, immunofluorescence ou immunoprécipitation avec des anticorps spécifiques, etc.).

D'autres procédés plus simples ou plus complexes, conduisant à des produits moins chers ou plus purs sont facilement envisageables par l'homme de l'art connaissant le métier d'extraction et de purification des protéines et peptides.

A titre d'exemple illustrant l'invention, on cite quelques formules cosmétiques représentatives mais non limitatives de l'invention:

### Exemple n° 1: Gel

| | |
|---|---|
| Carbopol 1342^{R} | 0,3 |
| Propylène glycol | 2,0 |
| Glycérine | 1,0 |
| Vaseline blanche | 1,5 |
| Cylomethicone | 6,0 |
| Alcool cétylique | 0,5 |
| Lubrajel^{R} MS | 10 |
| triéthanolamine | 0,3 |
| N-Palmitoyl-Sarcosine-Lys-Thr-Thr-Lys-Ser | 0,0005 |
| Eau, conservateurs, parfum | qsp 100 g. |

### Exemple n°2: Crème

| | |
|---|---|
| Volpo S20 | 2.4 |
| Volpo S2 | 2.6 |
| Prostéaryl 15 | 8.0 |
| Cire d'abeille | 0.5 |
| Abil^{R} ZP 2434 | 3.0 |
| Propylène glycol | 3.0 |
| Carbopol^{R} 941 | 0.25 |
| Triéthanolamine | 0.25 |
| N-Palmitoyl-Lys-Thr-Thr-Lys-Ser | 0,005 |
| Eau, conservateurs, parfums qsp | 100 g |

Les activités décrites au début de cette demande sont illustrées par les exemples suivants.

### Exemple n° 3: Augmentation de la synthèse de collagène: in vitro

La méthode choisie est une variante de celle décrite par Augustin C. et al. (*Skin Pharmacol.* (1997);**10**:63-70) en ce que nous avons utilisé des explants de peau humaine au lieu de fibroblastes pulmonaires humains, ceci afin de rendre nos résultats directement exploitables en cosmétologie.

Ces explants, provenant de plastie mammaire ou abdominale, sont incubés, pendant 72 heures en présence de ³H-proline, avec le peptide, N-Palmitoyl-Sarcosine-Thr-Thr-Lys-Ser, sous trois concentrations finales dans le milieu de culture (2.10⁻⁴ %, 4.10⁻⁴ % et 8.10⁻⁴ %; soit 2, 4 et 8 ppm). Les explants sont alors lavés, le derme et l'épiderme de chaque explant sont séparés, homogénéisés et lysés. La mesure de l'incorporation de ³H-proline est alors réalisée dans chaque lysat. Les essais sont faits en triplicate.

Parallèlement des contrôles *négatifs* sont réalisés dans le mêmes conditions mais en l'absence du peptide. Des contrôles, *positifs,* quant à eux, sont réalisés en remplaçant le peptide testé par de la vitamine C.

En présence de 2, 4 ou 8 ppm de peptide, l'incorporation de ³H-proline qui traduit la synthèse de collagène, est augmentée de respectivement 30,2 (± 2) %, 54,7 (± 5) % et 90,9 (± 5) % par rapport à ce qui est constaté dans les expériences témoin (sans le peptide).

Dans les mêmes conditions, le produit de référence, l'acide ascorbique, à la concentration de 0.5mM, augmente la synthèse du collagène de 61,4 (± 5) %.

### Exemple n° 4: Augmentation de la synthèse de glycosaminoglycanes: in vitro

Le même protocole que celui de l'exemple n°3 est utilisé, si ce n'est que, d'une part, l'incubation est réalisée en présence de ³H-glucosamine à la place de la ³H-proline et que, d'autre part, la vitamine A acide est utilisée comme produit de référence à la place de la vitamine C.

En présence de 2, 4 ou 8 ppm de peptide Lys-Thr-Thr-Lys-Ser-Ala, l'incorporation de ³H-glucosamine qui traduit la synthèse de GAGs, est augmentée de respectivement 24,5 (± 3) %, 48,8 (± 3) % et 67,9 (± 5) % par rapport à ce qui est constaté dans les expériences témoin (sans le peptide).

Dans les mêmes conditions, le produit de référence, la vitamine A acide, à la concentration de 100 nM, augmente la synthèse des GAGs de 45,3 (± 2) %.

Les résultats obtenus dans ces deux exemples démontrent clairement un effet, concentration dépendant, du peptide sur la synthèse des deux constituants de la matrice extracellulaire.

### Exemple n°5: Activité raffermissant cutané: in vitro

Durant 24 heures, des fibroblates humains provenant de la même culture cellulaire, sont mis en présence de milieu de culture standard supplémenté, ou non pour les contrôles, avec différentes concentrations de peptide (2, 4 et 8 ppm).

La stimulation de la synthèse de protéines est évaluée par colorimétrie (réaction dite du Biuret).

Pour standardiser les résultats, la quantité de protéines mesurée est exprimée pour 1000 cellules présentes dans le test.

Par rapport aux expérimentations contrôles, en présence soit de 2, 4 et 8 ppm de peptide N-palmytoyl-Lys-Thr-Thr-Lys-Ser, l'augmentation de la concentration de protéines est respectivement de 14,7 (± 1,0)%, 21,0 (±2,4)% et 44,8 (± 1,0)%.

Ainsi, cet essai *in vitro* démontre le potentiel stimulant, concentration dépendant, au niveau cutané du peptide, effet directement lié à un raffermissement et à un épaississement des peaux trop fines.

### Exemple n° 6: Activité antirides: in vivo

Cet exemple rapporte l'effet antirides obtenu, *in vivo,* sur un panel constitué de 15 volontaires adultes de sexe féminin, âgées de 35 à 63 ans. Le pouvoir antirides de la crème de l'exemple n°2, contenant le N-Palmitoyl-Lys-Thr-Thr-Lys-Ser à la concentration de 0,005 % (soit 50 ppm), est comparé à celui d'une crème placebo (même crème mais sans l'actif). Les crèmes sont appliquées sur des sites précisément identifiés, situés sur le coin de l'oeil droit ou gauche, selon une répartition randomisée, deux fois par jour, pendant 28 jours. Le paramètre pris en compte est le relief cutané, au niveau du contour de l'oeil (rides dites de la *patte d'oie).* Les quantifications des différentes variables du relief sont réalisées par analyse vidéo-informatique d'empreintes au silicone prises à la surface de la peau selon les protocoles décrits par Corcuff et al. (1985, *Int. J. Cosm. Sci.**7***:117-126) et Corcuff et al. (1995, in *Handbook of non-invasive methods and the skin,* Serup & Jemec eds., CRC Press:89-96).

Le tableau ci-dessous indique la différence, en pourcentage, des valeurs moyennes obtenues entre T +28 jours et T0 pour les profondeurs moyennes de la ride principale (colonne A) ou pour l'ensemble des plis (col. B); pour la densité des plis principaux (col. C) ainsi que pour la mesure de la rugosité (col. D).

| | **A** | **B** | **C** | **D** |
|---|---|---|---|---|
| **Placebo** | 0,2 | + 0,5 | - 1,1 | + 2,7 |
| **Peptide** | - 18,2 | - 21,1 | - 36,9 | - 21,3 |

La crème contenant le N-Palmitoyl-Lys-Thr-Thr-Lys-Ser précédemment décrit démontre clairement un puissant effet antirides puisque l'on observe une différence importante entre le début et la fin de l'étude *in vivo* et ceci, sur l'ensemble des quatre paramètres classiquement utilisés dans cette indication.

Il est à noter que, dans les mêmes conditions expérimentales, la crème placebo ne présente aucun effet si le N-Palmitoyl-Lys-Thr-Thr-Lys-Ser n'y a pas été incorporé, ce qui démontre bien que c'est seulement au peptide faisant l'objet de cc brevet que l'on peut attribuer l'effet bénéfique observé.

Il est particulièrement avantageux d'utiliser ces peptides en combinaison entre eux.

Les peptides de ce brevet peuvent être obtenus par synthèse chimique, par voie enzymatique, par fermentation, par extraction de protéines d'origine végétale, par synthèse peptidique classique en phase homogène ou hétérogène ou par synthèse enzymatique à partir des acides aminés constitutifs.

Les peptides de ce brevet peuvent être obtenus par extraction de protéines de plantes, suivie d'hydrolyse, enzymatique ou non enzymatique, de façon à générer des fragments peptidiques de taille moyenne comprise entre 300 et 2000 daltons, une partie des fragments libérés devant contenir la séquence X-Thr-Thr-Lys-(AA)ₙ, préférentiellement la séquence Lys-Thr-Thr-Lys-Scr.

Les peptides de ce brevet, seuls ou en association entre eux, peuvent être utilisés à des concentrations variant entre 0,1 et 1000 ppm (p/p), préférentiellement entre 1 et 100 ppm (p/p) dans le produit cosmétique ou dermopharmaceutique fini.

Les peptides de ce brevet, seuls ou en association entre eux, peuvent être utilisés sous forme de solution, de dispersion, d'émulsion, ou encapsulé dans des vecteurs comme les macro-, micro- ou nanocapsules, des liposomes ou des chylomicrons, ou inclus dans des macro-, micro- ou nanoparticules, ou dans des microéponges, ou adsorbé sur des polymères organiques poudreux, les talcs, bentonites et autres supports minéraux.

Les peptides de ce brevet, seuls ou en association entre eux, peuvent être utilisés dans toute forme galénique: émulsions H/E et E/H, laits, lotions, polymères gélifiants et viscosants, tensioactifs et émulsifiants, pommades, lotions capillaires, shampooings, savons, poudres, sticks et crayons, sprays, huiles corporelles.

Les peptides de ce brevet, seuls ou en association entre eux, peuvent être utilisés avec tout autre ingrédient habituellement utilisé: lipides d'extraction et/ou de synthèse, polymères gélifiants et viscosants, tensioactifs et émulsifiants, principes actifs hydro- ou liposolubles, extraits végétaux, extraits tissulaires, extraits marins, filtres solaires, antioxydants.

Les peptides de ce brevet, seuls ou en association entre eux, sont utilisés pour la préparation de compositions cosmétiques ou dermopharmaceutiques, ou pour la préparation de médicaments en vue de la synthèse concomittante de collagène et de glycosaminoglycanes.

Les peptides de ce brevet, seuls ou en association entre eux, sont utilisés pour favoriser l'hydratation, et pour tous les soins de la peau, particulièrement contre la formation et contre l'aggravation des rides et contre toutes les conséquences du vieillissement cutané, naturel ou accéléré (héliodermie, pollution).

Les peptides de ce brevet, seuls ou en association entre eux, sont utilisés pour la préparation d'un médicament pour favoriser la régénération cutanée et la cicatrisation.

## Revendications

1. Peptides de formule générale R₁-X-Thr-Thr-Lys-(AA)ₙ-Y et leurs sels avec :
• X représentant un acide aminé basique de forme D ou L (lysine, arginine, histidine, ornithine, citrulline, sarcosine, statine),
• (AA)ₙ représentant un enchaînement de n acides aminés naturels ou non, avec n variant de 0 à 5,
• R₁ étant H ou une chaîne d'acide gras, de 2 à 22 carbones, hydroxylée ou non, saturée ou non, linéaire ou ramifiée, soufrée ou non, cyclique ou non, ou un groupement biotinyl, ou un groupement protecteur de type uréthane utilisé en synthèse peptidique tel que les groupements benzyloxycarbonyl (Z), terbutyloxycarbonyl (tBoc), fluorenylméthyloxycarbonyl (Fmoc), allyloxycarbonyl (Alloc)
• Y = OR₂ ou NR₂R₃ avec R₂ et/ou R₃ étant un atome d'hydrogène H ou une chaîne aliphatique ou aromatique de 1 à 22 carbones, hydroxylée ou non, saturée ou non, linéaire ou ramifiée, soufrée ou non, cyclique ou non,
à l'exception des peptides avec R₁ = H et X = Lys et Y = OH et avec n = 0 ou (AA)ₙ = Ser quand n = 1.

2. Peptides selon la revendication 1 **caractérisés en ce que** n = 1, R₁ est une chaîne d'acide gras de 2 à 22 carbones et Y est OH ou NH₂.

3. Peptide selon la revendication 2 **caractérisé en ce que** X est la lysine, (AA)ₙ est la sérine, R₁ est le groupement palmitoyl et Y est le groupement OH.

4. Compositions cosmétiques ou dermopharmaceutiques renfermant un (des) peptide(s) selon les revendications 1 à 3.

5. Compositions cosmétiques ou dermopharmaceutiques selon la revendication 4, **caractérisées en ce que** le ou les peptide(s), est/sont obtenu(s) par synthèse chimique, par voie enzymatique, par fermentation ou par extraction de protéines d'origine végétale.

6. Compositions cosmétiques ou dermopharmaceutiques selon les revendications 4 et 5, **caractérisées en ce que** le ou les peptide(s) est/sont obtenu(s) par synthèse peptidique classique en phase homogène ou hétérogène ou par synthèse enzymatique à partir des acides aminés constitutifs ou de leurs dérivés.

7. Compositions cosmétiques ou dermopharmaceutiques selon les revendications 4 à 6 **caractérisées en ce que** le ou les peptide(s) est/sont obtenu(s) par extraction de protéines de plantes, suivie d'hydrolyse, enzymatique ou non enzymatique, de façon à générer des fragments peptidiques de taille moyenne comprise entre 300 et 2000 daltons, une partie des fragments libérés devant contenir la séquence X-Thr-Thr-Lys-(AA)ₙ, préférentiellement la séquence Lys-Thr-Thr-Lys-Ser, à l'exclusion des fragments libérés contenant exclusivement le peptide avec R1=H et X=Lys et Y=OH et avec n=0 ou (AA)ₙ=Ser quand n=1.

8. Compositions cosmétiques ou dermopharmaceutiques selon les revendications 4 à 7 **caractérisées en ce que** le ou les peptide(s) est/sont utilisé(s) à des concentrations variant entre 0,1 et 1000 ppm (p/p), préférentiellement entre 1 et 100 ppm (p/p) dans le produit fini.

9. Compositions cosmétiques ou dermopharmaceutiques selon les revendications 4 à 8 **caractérisées en ce que** le ou les peptide(s) est/sont utilisé(s) sous forme de solution, de dispersion, d'émulsion, ou encapsulé dans des vecteurs comme les macro-, micro- ou nanocapsules, des liposomes ou des chylomicrons, ou inclus dans des macro-, micro- ou nanoparticules, ou dans des microéponges, ou adsorbé sur des polymères organiques poudreux, les talcs, bentonites et autres supports minéraux.

10. Compositions cosmétiques ou dermopharmaceutiques selon les revendications 4 à 9 **caractérisées en ce que** le ou les peptide(s) est/sont utilisé(s) dans toute forme galénique : émulsions H/E et E/H, laits, lotions, polymères gélifiants et viscosants, tensioactifs et émulsifiants, pommades, lotions capillaires, shampooings, savons, poudres, sticks et crayons, sprays, huiles corporelles.

11. Compositions cosmétiques ou dermopharmaceutiques selon les revendications 4 à 10 **caractérisées en ce que** le ou les peptide(s) est/sont utilisé(s) avec tout autre ingrédient habituellement utilisé : lipides d'extraction et/ou de synthèse, polymères gélifiants et viscosants, tensioactifs et émulsifiants, principes actifs hydro- ou liposolubles, extraits végétaux, extraits tissulaires, extraits marins, filtres solaires, antioxydants.

12. Compositions cosmétiques ou dermopharmaceutiques selon les revendications 4 à 11 utilisées dans les applications cosmétiques pour favoriser la cicatrisation, l'hydratation, et pour tous les soins de la peau, particulièrement contre la formation et l'aggravation des rides ainsi que contre toutes les conséquences du vieillissement cutané, naturel ou accéléré (héliodermie, pollution).

13. Utilisation de peptide(s) selon les revendications 1 à 3 pour la préparation de compositions cosmétiques ou dermopharmaceutiques en vue de la synthèse concomitante de collagène et de glycosaminoglycanes.

14. Utilisation de peptides selon les revendications 1 à 3 pour la préparation de médicament en vue de la synthèse concomitante de collagène et de glycosaminoglycanes.

15. Utilisation de peptide(s) selon les revendications 1 à 3, pour la préparation de compositions cosmétiques pour favoriser l'hydratation, et pour tous les soins de la peau, particulièrement contre la formation et l'aggravation des rides ainsi que contre toutes les conséquences du vieillissement cutané, naturel ou accéléré (héliodermie, pollution).

16. Utilisation de peptide(s) selon les revendications 1 à 3, pour la préparation d'un médicament pour favoriser la régénération cutanée et la cicatrisation.

## Claims

1. Peptides of the general formula R₁-X-Thr-Thr-Lys-(AA)ₙ-Y, and their salts wherein:
• X represents a basic amino acid of D or L orientation (lysine, arginine, histidine, ornithine, citruline, sarcosine, statine),
• (AA)ₙ represents a chain of n amino acids, natural or not, with n varying from 0 to 5,
• R₁ be H or a fatty acid chain of 2 to 22 carbons, hydroxylated or not, saturated or not, linear or branched, sulfurated or not, cyclic or not, or a biotin group, or a protective group of the urethane type used in peptide synthesis such as the groups benzyloxycarbonyl (Z), terbutyloxycarbonyl (tBoc), fluorenylmethyloxycarbonyl (Fmoc), allyloxycarbonyl (Alloc),
• Y = OR₂ or NR₂R₃ wherein R₂ and/or R₃ are a hydrogen atom or an aliphatic or aromatic chain of 1 to 22 carbons, hydroxylated or not, saturated or not, linear or branched, sulfurated or not, cyclic or not,
except peptides wherein R₁ =H and X=Lys and Y=OH and with n=0 or (AA)ₙ =Ser when n=1,

2. The peptides according to claim 1, wherein n=1, R₁ is a fatty acid chain of 2 to 22 carbons, and Y is OH or NH₂.

3. The peptide according to claim 2, wherein X is lysine, (AA)ₙ is serine, R₁ is the palmitoyl group, and Y is the OH group.

4. Cosmetic or dermopharmaceutical compositions including a peptide or peptides according to claims 1 to 3.

5. Cosmetic or dermopharmaceutical compositions according to claim 4, wherein the peptide(s) is/are obtained by chemical synthesis, by fermentation, or by extraction of proteins of vegetable origin.

6. Cosmetic or dermopharmaceutical compositions according to claims 4 and 5, wherein the peptide(s) is/are obtained by conventional peptide synthesis in homogeneous or heterogeneous phase or by enzymatic synthesis from constituent amino acids or their derivatives.

7. Cosmetic or dermopharmaceutical compositions according to claims 4 to 6, wherein the peptide(s) is/are obtained by extraction of plant proteins, followed by hydrolysis, enzymatic or non-enzymatic, so as to generate peptide fragments of a mean size comprised between 300 and 2000 daltons, a portion of the freed fragments containing the sequence X-Thr-Thr-Lys-(AA)ₙ, preferably the sequence Lys-Thr-Thr-Lys-Ser, except the freed fragments comprising exclusively the peptide with R₁ = H and X = Lys, and Y = OH and with n = 0 or (AA)ₙ = Ser when n = 1.

8. Cosmetic or dermopharmaceutical compositions according to claims 4 to 7, wherein the peptide(s) is/are used at concentrations varying between 0.1 and 1000 ppm by weight, preferentially between 1 and 100 ppm by weight based on the total weight of said composition.

9. Cosmetic or dermopharmaceutical compositions according to claims 4 to 8, wherein the peptide(s) is/are used in the form of a solution, dispersion, emulsion, or encapsulated in vectors such as macro-, micro- or nano-capsules, lyposomes or chylomicrons, or included in macro-, micro- or nano-particles, or in micro-sponges, or adsorbed on powdered organic polymers, talcs, bentonites and other mineral supports.

10. Cosmetic or dermopharmaceutical compositions according to claims 4 to 9, wherein the peptide(s) is/are used in any galenic form: O/W emulsions and W/O emulsions, milks, lotions, gelifying and thickening, tensioactive and emulsifying polymers, pomades, hair lotions, shampoos, soaps, powders, sticks and pencils, sprays, body oils.

11. Cosmetic or dermopharmaceutical compositions according to claims 4 to 10, wherein the peptide(s) is/are used with any other ingredient conventionally used: extraction and/or synthesis lipids, gelifying and thickening, tensioactive and emulsifying polymers, hydrosoluble or liposoluble active principles, vegetable extracts, tissue extracts, marine extracts, solar filters, antioxidants.

12. Cosmetic or dermopharmaceutical compositions according to claims 4 to 11 used in cosmetic applications to promote healing, hydration and for all skin care, particularly against the formation and worsening of wrinkles and against any consequences of cutaneous aging, natural or accelerated (heliodermia, pollution).

13. Use of peptide(s) according to claims 1 to 3 for the preparation of cosmetic or dermopharmaceutical compositions for the concomitant synthesis of collagen and glycosaminoglycans.

14. Use of peptide(s) according to claims 1 to 3 for the preparation of medication for the concomitant synthesis of collagen and glycosaminoglycans.

15. Use of peptide(s) according to claims 1 to 3, for the preparation of cosmetic compositions to promote healing, hydration and for all skin care, particularly against the formation and worsening of wrinkles and against any consequences of cutaneous aging, natural or accelerated (heliodermia, pollution).

16. Use of peptide(s) according to claims 1 to 3 for the preparation of medication for the skin regeneration and healing.

## Patentansprüche

1. Peptide der allgemeinen Formel R₁-X-Thr-Thr-Lys-(AA)ₙ-Y und ihre Salze, wobei:
- X eine basische Aminosäure der Form D oder L (Lysin, Arginin, Histidin, Omithin, Zitrullin, Sarcosin, Statin) repräsentiert,
- (AA)ₙ eine Verkettung von n natürlichen oder nichtnatürlichen Aminosäuren darstellt, wobei n von 0 bis 5 variiert,
- R₁ für H steht oder für eine Fettsäurekette mit 2 bis 22 Kohlenstoffatomen, die hydroxyliert ist oder nicht, gesättigt ist oder nicht, linear oder verzweigt ist, geschwefelt ist oder nicht, zyklisch ist oder nicht, oder für eine Biotinylgruppe, oder für eine Schutzgruppe vom Urethantyp, die in der Peptidsynthese verwendet wird, wie z.B. die Gruppen Benzyloxycarbonyl (Z), Terbutyloxycarbonyl (tBoc), Fluorenylmethyloxycarbonyl (Fmoc), Allyloxycarbonyl (Alloc),
- Y = OR₂ oder NR₂R₃, wobei R₂ und/oder R₃ ein Wasserstoffatom oder eine aliphatische oder aromatische Kette mit 1 bis 22 Kohlenstoffatomen ist, die hydroxyliert ist oder nicht, gesättigt ist oder nicht, linear oder verzweigt ist, geschwefelt ist oder nicht, zyklisch ist oder nicht,
mit Ausnahme der Peptide mit R₁ = H und X = Lys und Y = OH sowie mit n = 0 oder (AA)ₙ = Ser n = 1.

2. Peptide nach Anspruch 1, ***dadurch gekennzeichnet,* dass** n = 1, R₁ eine Fettsäurekette mit 2 bis 22 Kohlenstoffatomen ist und Y für OH oder NH₂ steht.

3. Peptide nach Anspruch 2, ***dadurch gekennzeichnet,* dass** X das Lysin, (AA)ₙ das Serin, R₁ die Palmitoylgruppe und Y die Gruppe OH ist.

4. Kosmetische oder dermopharmazeutische Zusammensetzungen, die ein Peptid oder Peptide gemäß den Ansprüchen 1 bis 3 enthalten.

5. Kosmetische oder dermopharmazeutische Zusammensetzungen gemäß Anspruch 4, ***dadurch gekennzeichnet,* dass** das Peptid oder die Peptide durch chemische Synthese, auf enzymatischen Weg, durch Fermentierung oder Extraktion von Proteinen pflanzlichen Ursprungs erhalten wird (werden).

6. Kosmetische oder dermopharmazeutische Zusammensetzungen gemäß den Ansprüchen 4 und 5, ***dadurch gekennzeichnet,* dass** das Peptid oder die Peptide durch klassische Peptidsynthese in homogener oder heterogener Phase oder durch enzymatische Synthese, ausgehend von den Bestandteils-Aminosäuren oder ihren Derivaten, erhalten wird (werden).

7. Kosmetische oder dermopharmazeutische Zusammensetzungen nach den Ansprüchen 4 bis 6, ***dadurch gekennzeichnet,* dass** das Peptid oder die Peptide durch Extraktion von pflanzlichen Proteinen, gefolgt von enzymatischer oder nichtenzymatischer Hydrolyse erhalten werden, derart, dass Peptidfragmente von einer mittleren Größe zwischen 300 und 2000 Daltons erhalten werden, wobei ein Teil der freigesetzten Fragmente die Sequenz X - Thr-Thr-Lys-(AA)ₙ, vorzugsweise die Sequenz Lys-Thr-Thr-Lys-Ser, enthalten muss, mit Ausnahme von freigesetzten Fragmenten, die ausschließlich das Peptid mit R₁ = H und X = Lys sowie Y = OH und mit n = 0 oder (AA)ₙ = Ser, wenn n = 1 ist, enthalten.

8. Kosmetische oder dermopharmazeutische Zusammensetzungen gemäß den Ansprüchen 4 bis 7, ***dadurch gekennzeichnet,* dass** das Peptid oder die Peptide in dem Fertigprodukt in Konzentrationen verwendet wird (werden), die zwischen 0,1 und 1000 ppm (p/p) liegen, vorzugsweise zwischen 1 und 100 ppm (p/p).

9. Kosmetische oder dermopharmazeutische Zusammensetzungen gemäß den Ansprüchen 4 bis 8, ***dadurch gekennzeichnet,* dass** das Peptid oder die Peptide verwendet werden in Form einer Lösung, einer Dispersion, einer Emulsion oder verkapselt in Trägem wie Makro-, Mikro- oder Nanokapseln, Liposomen oder Chylomikrons, oder enthalten in Makro-, Mikro oder Nanopartikeln, oder in Mikroschwämmen, oder adsorbiert auf organischen pulverförmigen Polymeren, Talk oder Bentoniten oder anderen mineralischen Trägem.

10. Kosmetische oder dermopharmazeutische Zusammensetzungen gemäß den Ansprüchen 4 bis 9, ***dadurch gekennzeichnet,* dass** das Peptid oder die Peptide in beliebiger galenischer Form verwendet wird (werden): H/E- und E/H-Emulsionen, Milche, Lotionen, gelbildende und viskose Polymere, Tensid und Emulgatoren, Pomaden, Kappilarlotionen, Shampoos, Seifen, Puder, Stifte, Sprays, Körperölen.

11. Kosmetische oder dermopharmazeutische Zusammensetzungen gemäß den Ansprüchen 4 bis 10, ***dadurch gekennzeichnet,* dass** das Peptid oder die Peptide verwendet wird (werden) mit beliebigen anderen üblicherweise verwendeten Ingredienzien: Extraktions- und/oder Syntheselipide, gelbildende und viskose Polymere, Tenside und Emulgatoren, wasser- oder fettlöslichen Wirkstoffen, Pflanzenextrakten, Gewebeextrakten, Meeresextrakten, Sonnenfiltern, Antioxidanzien.

12. Kosmetische oder dermopharmazeutische Zusammensetzungen gemäß den Ansprüchen 4 bis 11, die verwendet werden in kosmetischen Anwendungen zur Begünstigung der Narbenbildung, der Feuchtigkeitszufuhr und für alle Hautpflegeanwendungen, insbesondere gegen die Bildung und Verschlimmerung von Falten sowie gegen alle Folgen der natürlichen oder beschleunigten Hautalterung (Heliodermie, Umweltverschmutzung).

13. Verwendung des Peptids (der Peptide) gemäß den Ansprüchen 1 bis 3 für die Zubereitung von kosmetischen oder dermopharmazeutischen Zusammensetzungen im Hinblick auf die begleitende Synthese von Kollagen und von Glykosaminoglykanen.

14. Verwendung von Peptiden gemäß den Ansprüchen 1 bis 3 für die Zubereitung von Medikamenten im Hinblick auf die begleitende Synthese von Kollagen und von Glykosaminoglykanen.

15. Verwendung des Peptids (der Peptide) gemäß den Ansprüchen 1 bis 3 für die Zubereitung von kosmetischen Zusammensetzungen zur Begünstigung der Feuchtigkeitszufuhr, und für alle Hautpflegeanwendungen, insbesondere gegen die Bildung und Verschlimmerung von Falten sowie gegen alle Folgen der natürlichen oder beschleunigten Hautalterung (Heliodermie, Umweltverschmutzung).

16. Verwendung des Peptids (der Peptide) gemäß den Ansprüchen 1 bis 3 für die Zubereitung eines Medikaments zum Fördern der Hautregeneration und der Narbenbildung.
